(19) Europäisches Patentamt
European Patent Office
Office européen des brevets

(11) Veröffentlichungsnummer: **0 562 530 A1**

(12) **EUROPÄISCHE PATENTANMELDUNG**

(21) Anmeldenummer: **93104703.9**

(22) Anmeldetag: **22.03.93**

(51) Int. Cl.5: **C07C 43/305**, C07D 493/10, C07C 49/707, C07C 49/39, C07C 41/56, C07C 45/42, C07C 45/85, //(C07D493/10, 317:00,317:00),(C07D493/10, 319:00,319:00)

(30) Priorität: **24.03.92 CH 929/92**

(43) Veröffentlichungstag der Anmeldung:
**29.09.93 Patentblatt 93/39**

(84) Benannte Vertragsstaaten:
**AT BE CH DE DK ES FR GB IE IT LI LU NL SE**

(71) Anmelder: **LONZA AG**
**Gampel/Wallis Geschäftsleitung Basel**
**CH-4002 Basel(CH)**

(72) Erfinder: **Jackson, Barry, Dr.**
**Jesuitenweg 158**
**Brig-Glis (Kanton Wallis)(CH)**

(74) Vertreter: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**D-80803 München (DE)**

(54) **1,3-Cyclobutandion,bisketale, Verfahren zu ihrer Herstellung und ihre Verwendung.**

(57) Beschrieben werden neue Bisketale des 1,3-Cyclobutandions mit einwertigen und zweiwertigen Alkoholen sowie Verfahren zu ihrer Herstellung aus 1,3-Cyclobutandion oder aus dem in Destillationsrückständender Diketen-Produktion enthaltenen 3-Acetoxy-2-cyclobuten-1-on.
Die Bisketale eignen sich als Zwischenprodukte zur Herstellung von Quadratsäure, zur Herstellung und/oder Reinigung von 1,3-Cyclobutandion, oder als stabile Lagerform oder Ersatz für dieses instabile Dion.

EP 0 562 530 A1

Die vorliegende Erfindung betrifft neue 1,3-Cyclobutandion-bisketale sowie Verfahren zu deren Herstellung und deren Verwendung, insbesondere zur Herstellung von Quadratsäure.

Quadratsäure (1,2-Dihydroxycyclobuten-3,4-dion) ist ein interessantes Zwischenprodukt zur Herstellung von Farbstoffen, Herbiziden und pharmazeutischen Wirkstoffen. Nachdem lange Zeit kein wirtschaftliches Herstellungsverfahren für Quadratsäure bekannt war, ist es inzwischen gelungen, Quadratsäure aus 3-Acetoxy-2-cyclobuten-1-on ("Triketen") oder dem daraus erhältlichen 1,3-Cyclobutandion bzw. dessen Enolaten herzustellen (EP-Anm. 442 431 und 444 563). Diese Verfahren erfordern jedoch im allgemeinen eine aufwendige Isolierung des Triketens oder des wenig stabilen 1,3-Cyclobutandions.

Aufgabe der vorliegenden Erfindung war es, stabile Derivate des 1,3-Cyclobutandions bereitzustellen, welche auch leicht aus dem bei der technischen Herstellung von Diketen aus Keten in grossen Mengen anfallenden triketenhaltigen Rückstand hergestellt und isoliert werden können, sowie deren Umsetzung zu Quadratsäure aufzuzeigen.

Erfindungsgemäss wurde diese Aufgabe durch die 1,3-Cyclobutandion-bisketale nach Patentanspruch 1 gelöst.

Die erfindungsgemässen 1,3-Cyclobutandion-bisketale haben die allgemeine Formel

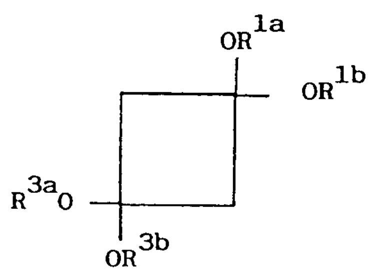

I

worin entweder $R^{1a}$, $R^{1b}$, $R^{3a}$ und $R^{3b}$ gleich sind und jeweils eine lineare oder verzweigte Niederalkylgruppe darstellen, oder jeweils $R^{1a}$ und $R^{1b}$ sowie $R^{3a}$ und $R^{3b}$ zusammen gleichbedeutend sind und mit den Ketal-Sauerstoffatomen und dem dazwischenliegenden Kohlenstoffatom jeweils einen, gegebenenfalls durch eine oder mehrere Niederalkylgruppen substituierten, fünfgliedrigen oder sechsgliedrigen mit dem Cyclobutanring spiro-verknüpften Ring bilden.

Der ersten Alternative entsprechen somit die Bisketale des 1,3-Cyclobutandions mit einwertigen Alkoholen, der zweiten Alternative diejenigen mit 1,2- bzw. 1,3-Diolen. Unter Niederalkylgruppen sind hier und im folgenden Alkylgruppen mit 1 bis 6, vorzugsweise 1 bis 4 Kohlenstoffatomen zu verstehen, also beispielsweise Methyl, Ethyl, Propyl, Isopropyl, Butyl, Isobutyl, sec-Butyl-, Pentyl, Isopentyl und Hexyl. Besonders bevorzugt sind die Bisketale, die sich von Methanol oder Ethanol ableiten, worin die Niederalkylgruppen also Methyl oder Ethyl sind. Unter den Bisketalen mit 1,2- und 1,3-Diolen sind diejenigen bevorzugt, in welchen $R^{1a}$ und $R^{1b}$ sowie $R^{3a}$ und $R^{3b}$ zusammen jeweils eine 1,2-Ethandiyl-, 1,2-Propandiyl-, 1,3-Propandiyl-, 1,2-Butandiyl-, 1,3-Butandiyl-, 2,3-Butandiyl-, 2,2-Dimethyl-1,2-propandiyl oder 2,3-Dimethyl-2,3-butandiyl-Gruppe bilden, sich also vom Ethandiol, 1,2-Propandiol, 1,3-Propandiol, 1,2-Butandiol, 1,3-Butandiol, 2,3-Butandiol, 2,2-Dimethyl-1,3-propandiol (Neopentylglykol) oder 2,3-Dimethyl-2,3-butandiol (Pinakol) ableiten.

Die erfindungsgemässen Bisketale können beispielsweise aus dem 1,3-Cyclobutandion durch Umsetzung mit dem entsprechenden Orthoameisensäureester in Gegenwart eines sauren Katalysators hergestellt werden. Die hierfür erforderlichen Orthoameisensäureester besitzen die allgemeine Formel

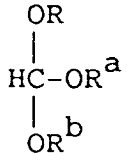

II

worin $R^a$ und $R^b$ die in Formel I für $R^{1a}$ und $R^{1b}$ sowie $R^{3a}$ und $R^{3b}$ genannten Bedeutungen haben und R eine Niederalkylgruppe ist. Sind $R^a$ und $R^b$ ebenfalls Niederalkylgruppen, handelt es sich also um ein Trialkylorthoformiat, so sind ausserdem R, $R^a$ und $R^b$ gleich. Die bevorzugten Orthoameisensäureester

ergeben sich aus den vorzugsweise herzustellenden Bisketalen I, besonders bevorzugt sind also Trimethylorthoformiat und Triethylorthoformiat. Entsprechend können aus cyclischen Orthoameisensäureestern, in welchen $R^a$ und $R^b$ zusammen mit den direkt gebundenen Sauerstoffatomen und dem dazwischenliegenden Formiat-Kohlenstoffatom einen, gegebenenfalls durch eine oder mehrere Niederalkylgruppen substituierten, fünfgliedrigen oder sechsgliedrigen Ring bilden, die entsprechenden Spiro-ketale hergestellt werden. Ein Beispiel für einen solchen Orthoameisensäureester ist das 2-Methoxy-1,3-dioxolan, das gemischte Orthoformiat von Ethandiol und Methanol.

Wird die Umsetzung mit einem Trialkylorthoformiat durchgeführt, gilt also $R = R^a = R^b$, so wird vorzugsweise in Gegenwart des entsprechenden Alkohols ROH, welcher gegebenenfalls gleichzeitig als Lösungsmittel dient, gearbeitet.

Als saurer Katalysator werden vorzugsweise saure Kationenaustauscher, beispielsweise Amberlyst[R]-15, eingesetzt.

Die Umsetzung des 1,3-Cyclobutandions mit den Orthoameisensäureester wird zweckmässig bei einer Temperatur von -10 bis +40 °C, vorzugsweise 0 bis 20 °C durchgeführt.

Der Orthoameisensäureester wird dabei vorteilhaft in einer Menge von 2 bis 10 mol, bezogen auf 1 mol 1,3-Cyclobutandion, eingesetzt.

Die erfindungsgemässen Bisketale sind thermisch stabil und können durch Destillation unter vermindertem Druck leicht abgetrennt und/oder gereinigt werden.

Ein weiteres erfindungsgemässes Verfahren zur Herstellung der erfindungsgemässen Bisketale, insbesondere derjenigen, welche sich von 1,2- bzw. 1,3-Diolen ableiten, umfasst zwei Stufen, wobei zunächst in der vorstehend beschriebenen Weise 1,3-Cyclobutandion mit einem Trialkylorthoformiat der allgemeinen Formel

$HC(OR)_3$ III

worin R eine Niederalkylgruppe ist, zu dem entsprechenden Bisketal der allgemeinen Formel

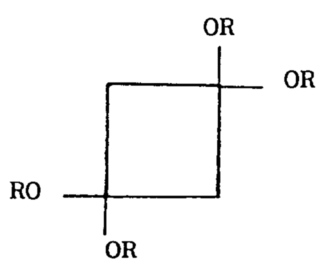

IV

umgesetzt und anschliessend mit einem Diol der allgemeinen Formel

HO-Q-OH V

worin Q eine gegebenenfalls durch eine oder mehrere Niederalkylgruppen substituierte Ethandiyl- oder Propandiylgruppe ist, in einer Umketalisierung in das entsprechende Spiroketal übergeführt wird.

Nach diesem Verfahren wird vorzugsweise zunächst mit Trimethyl- oder Triethylorthoformiat das Bis-dimethyl- bzw. Bis-diethylketal hergestellt.

Die Gruppe Q ist vorzugsweise 1,2-Ethandiyl, 1,2-Propandiyl, 1,3-Propandiyl, 1,2-Butandiyl, 2,3-Butandiyl, 2,2-Dimethyl-2,3-propandiyloder 2,3-Dimethyl-2,3-butandiyl.

Ein weiteres erfindungsgemässes Verfahren zur Herstellung der erfindungsgemässen Bisketale geht vom 3-Acetoxy-2-cyclobuten-1-on (Triketen) der Formel

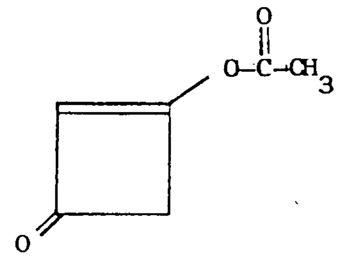

VI

aus. Hierbei handelt es sich um das Monoenolacetat des 1,3-Cyclobutandions. Das Triketen wird dazu zweckmässig in der vorstehenden für das 1,3-Cyclobutandion beschriebenen Weise mit einem Orthoameisensäureester umgesetzt. Vorteilhaft wird dabei kein reines Triketen, sondern der triketenhaltige Rückstand der technischen Diketen-Destillation eingesetzt.

Insbesondere bei der besonders bevorzugten Verwendung von Trimethylorthoformiat als Orthoameisensäureester kann dadurch das Triketen fast quantitativ aus dem Rückstand entfernt und verwertet werden. Die Isolierung des Bisketals kann dabei durch eine einfache Destillation unter vermindertem Druck, beispielsweise in einem Dünnschichtverdampfer, erfolgen.

Eine bevorzugte Verwendung der erfindungsgemässen 1,3-Cyclobutandion-bisketale ist die Verwendung als Ausgangsprodukt für die Herstellung von Quadratsäure. Hierzu werden die Ketale zunächst halogeniert und anschliessend die als Zwischenprodukt gebildeten Halogenverbindungen hydrolysiert.

Die Halogenierung wird vorteilhaft in Gegenwart von Wasser bzw. nach Zusatz von Wasser durchgeführt. Vermutlich werden dabei die Ketalfunktionen ganz oder teilweise hydrolysiert und die Hydrolyseprodukte in situ halogeniert.
Vorzugsweise wird die Halogenierung mit elementarem Chlor oder Brom durchgeführt, besonders bevorzugt mit Brom.
Das Halogen wird dabei zweckmässig in einer Menge von 2,5 bis 4 mol, vorzugsweise 3 bis 3,5 mol je mol Ausgangsmaterial eingesetzt.
Die Halogenierung wird zweckmässig bei einer Temperatur von 0 bis 80 °C, vorzugsweise von 10 bis 40 °C durchgeführt. Als Lösungsmittel eignen sich alle gegen elementares Halogen beständigen protischen und aprotischen Lösungsmittel, insbesondere niedere aliphatische Carbonsäuren, deren Ester und Anhydride, sowie halogenierte Alkane. Als Beispiel für diese Lösungsmittel seien hier Essigsäure, Ethylacetat, Essigsäureanhydrid, Dichlormethan, Trichlormethan und Tetrachlormethan genannt. Besonders bevorzugt als Lösungsmittel ist Essigsäure.

Die Hydrolyse zur Quadratsäure wird vorzugsweise mit Schwefelsäure durchgeführt, welche die erforderliche Menge Wasser (2 mol für 1 mol Quadratsäure) oder einen Überschuss an Wasser enthält. Es ist auch möglich, die Hydrolyse mit Wasser allein oder mit anderen wässrigen Säuren durchzuführen.
Beispiele für solche Säuren sind Mineralsäuren wie Salzsäure, Bromwasserstoffsäure, wässrige Phosphorsäure, starke Carbonsäuren wie wässrige Ameisensäure oder wässrige Trifluoressigsäure, oder wässrige Sulfonsäuren wie Methansulfonsäure oder p-Toluolsulfonsäure.
Die Hydrolyse wird zweckmässig bei einer Temperatur von 50 bis 150 °C durchgeführt, vorzugsweise bei 80 bis 120 °C.

Ein weitere bevorzugte Verwendung der erfindungsgemässen 1,3-Cyclobutandionbisketale ist die Verwendung zur Herstellung und/oder Reinigung von 1,3-Cyclobutandion. Zu diesem Zweck werden die Bisketale in Gegenwart einer Säure hydrolysiert. Diese Verwendung ist insbesondere für die Herstellung von 1,3-Cyclobutandion aus dem triketenhaltigen Diketen-Destillationsrückstand von Interesse, weil hierdurch die Isolierung und Reinigung des Triketens umgangen werden kann.

Wegen ihrer leichten Überführbarkeit in 1,3-Cyclobutandion und ihrer Stabilität eignen sich die erfindungsgemässe Bisketale auch ganz allgemein als Lagerform des instabilen 1,3-Cyclobutandions.

Die nachfolgenden Beispiele verdeutlichen die Ausführung der Erfindung.

Beispiel 1:

1,1,3,3-Tetramethoxycyclobutan aus 1,3-Cyclobutandion

In 25 ml Methanol wurden 3,12 g 1,3-Cyclobutandion (Gehalt 94,2%, 33 mmol) gelöst und auf 0 °C abgekühlt.
Die Lösung wurde mit 0,78 g Amberlyst$^{R}$-15 und anschliessend innerhalb von 20 min tropfenweise mit 37,9

g Trimethylorthoformiat (98%ig, 350 mmol) versetzt.
Nach weiteren 16 h Rühren bei 0°C wurde der Katalysator abfiltriert und das Filtrat bei 30°C/15 mbar eingeengt. Der Rückstand (5,9 g) wurde im Kugelrohr-Apparat bei 55 - 65°C/0,8 mbar destilliert.

| | |
|---|---|
| Ausbeute: | 4,79 g, Gehalt (GC) 98,4%, entspr. 76,4% d. Th. |
| $^1$H-NMR: ($CDCl_3$, 300 MHz) $\delta$ | 3,19 (s, 12H), 2,34 (s, 4H) |
| MS: m/z(%) | 161(8, M-15), 145(11), 113(8), 88(50), 71(5), 58(31), 43(100) |

Beispiel 2:

1,1,3,3-Tetraethoxycyclobutan aus 1,3-Cyclobutandion

In 25 ml Ethanol wurden 3,12 g 1,3-Cyclobutandion (Gehalt 94,2%, 35 mmol) gelöst und mit 0,78 g Amberlyst$^R$-15 und 52,9 g Triethylorthoformiat (98%ig, 350 mmol) 24 h bei Raumtemperatur gerührt . Anschliessend wurde der Katalysator abfiltriert und das Filtrat bei 20°C/0,2 mbar eingeengt. Der Rückstand (8,1 g) wurde im Kugelrohr-Apparat bei 60 - 70°C/0,15 mbar destilliert.

| | |
|---|---|
| Ausbeute: | 7,22 g, Gehalt (GC) 94,7 g, entspr. 84,1 d. Th. |
| $^1$H-NMR: ($CDCl_3$, 300 MHz) $\delta$ | 3,44 (q, 8H), 2,38 (s, 4H), 1,21 (t, 12H) |
| MS: m/z(%): | 203(5, M-29), 187(5), 129(11), 116(15), 101(8), 89(28), 85(27), 60(25), 43(100). |

Beispiel 3:

1,1,3,3-Tetrabutoxycyclobutan aus 1,3-Cyclobutandion

In 15 ml Butanol wurden 1,77 g 1,3-Cyclobutandion (Gehalt 95,0%, 20 mmol) gelöst und mit 0,44 g Amberlyst$^R$-15 und 10,33 g Tributylorthoformiat (99%ig, 44 mmol) 7 h bei 25°C gerührt. Anschliessend wurde der Katalysator abfiltriert und das Filtrat bei 50°C/15 mbar eingeengt. Der Rückstand (7,7 g wurde im Kugelrohr-Apparat bei 120 - 135°C/0,03 mbar destilliert.

| | |
|---|---|
| Ausbeute: | 3,87 g, Gehalt (GC) 97,0%, entspr. 54,5% d. Th. |
| $^1$H-NMR: ($CDCl_3$, 300 MHz), $\delta$ | 3,35 (t, 8H), 2,34 (s, 4H), 1,54 (m, 8H), 1,49 (m, 8H), 0,93 (t, 12H). |
| MS: m/z (%): | 287(9, M-57), 271(17), 231(10), 215(1), 175(2), 157(54), 117(40), 103-(60), 101(65), 85(22), 61(100), 56(64), 43(23), 41(59). |

Beispiel 4:

1,1,3,3-Tetramethoxycyclobutan aus dem Diketen-Destillationsrückstand

In 250 g Methanol wurden 250 g Diketen-Rückstand mit einem Gehalt von 52,5 g (0,416 mol) 3-Acetoxy-2-cyclobuten-1-on (Triketen) und 15,0 g (0,178 mol) Diketen gelöst und auf 0°C abgekühlt. Anschliessend wurden 12,49 g Amberlyst$^R$-15 und 113,7 g Trimethylorthoformiat (98%ig, 1,05 mol) zugegeben und die Mischung 20 h bei 10°C gerührt. Der Katalysator wurde abfiltriert und das Filtrat bei 20°C/5 mbar eingeengt. Der Rückstand (235,7 g) wurde in einem SAMBAY-Dünnschichtverdampfer bei 100°C/1 - 2 mbar destilliert.
Ausbeute: 89,9 g, Gehalt (GC) 77,1%, entspr. 94,6% d. Theorie, bezogen auf das 3-Acetoxy-2-cyclobuten-1-on im Ausgangsmaterial.

Beispiel 5:

1,4,8,11-Tetraoxadispiro[4.1.4.1]dodecan

In 19,7 g Ethandiol wurden 4,56 g 1,1,3,3-Tetramethoxycyclobutan (Gehalt 96,7%, 25 mmol) auf 135°C erhitzt. Dabei wurde zunächst bei Normaldruck die Haupttrennung des entstehenden Methanols und anschliessend (nach 2 h) bei 200 mbar der Rest der leichtflüchtigen Bestandteile abdestilliert. Aus dem Rückstand kristallisierte beim Abkühlen auf Raumtemperatur das Produkt aus, das abfiltriert und bei 20°C/0,005 mbar getrocknet wurde.

| | |
|---|---|
| Ausbeute: | 2,11 g, Gehalt (GC) 82,8% entspr. 40,6% d. Th. |
| Schmp.: | 95 - 103°C |
| $^1$H-NMR ($CDCl_3$ 300 MHz) $\delta$ | 3,93 (s, 8H), 2,69 (s, 4H) |

| | |
|---|---|
| MS: m/z (%) | 173(0,1, M+1 ), 141(2), 112(3), 100(23), 86(100), 68(7), 56(3), 55(4), 42-(94). |

Beispiel 6:

1,5,9,13-Tetraoxadispiro[5.1.5.1]tetradecan

In 23,5 g 1,3-Propandiol wurden 4,56 g 1,1,3,3-Tetramethoxycyclobutan (Gehalt 96,7%, 25 mmol) auf 140 °C erhitzt. Das entstehende Methanol wurde innerhalb von 6 h bei 100 mbar abdestilliert und das Reaktionsgemisch anschliessend auf Raumtemperatur abgekühlt, wobei das Produkt auskristallisierte. Die Kristalle wurden abfiltriert und bei 70 °C/0,02 mbar getrocknet.

| | |
|---|---|
| Ausbeute: | 2,10 g, Gehalt (GC) 87,4% entspr. 36,6% d. Th. |
| Schmp.: | 147 - 151 °C |
| $^1$H-NMR ($CDCl_3$, 300 MHz) δ | 3,86 (t, 8H), 2,53 (s, 4H), 1,7 (quint, 4H). |
| MS: m/z (%) | 201(0,5, M+1), 172(2), 157(6), 140(1), 113(25), 100(100), 72(18), 70(14), 57(9), 42(75). |

Beispiel 7:

3,3-11,11-Tetramethyl-1,5,9,13-tetraoxadispiro[5.1.5.1)tetradecan

31,88 g 2,2-Dimethyl-1,3-propandiol wurden mit 4,56 g 1,1,3,3-Tetramethoxycyclobutan (Gehalt 96,7%, 25 mmol) auf 140 °C erhitzt. Innerhalb von 2 h wurde das entstehende Methanol bei 200 mbar abdestilliert. Anschliessend wurde in einer Sublimationsapparatur bei 140 °C/50 mbar das überschüssige Diol entfernt, wobei das Produkt als Rückstand blieb.

| | |
|---|---|
| Ausbeute: | 3,95 g, Gehalt (GC) 93,5%, entspr. 52,4% d. Th. |
| Schmp.: | 166 - 171 °C |
| $^1$H-NMR ($CDCl_3$, 300 MHz) δ | 3,44 (s, 8H), 2,52 (s, 4H), 0,98 (s, 12H) |
| MS: m/z (%) | 257 (1, M+1), 241(5), 200(2), 186(5), 171(2), 141(32), 128(100) 86(11), 85(11), 69(90), 57(38), 55(32), 43(49), 42(36), 41(70). |

Beispiel 8:

Quadratsäure

In 1000 ml Essigsäure mit 36,6 g Wasser wurden 104,6 g 1,1,3,3-Tetramethoxycyclobutan (Gehalt 85,9%, 0,50 mol) gelöst. Die Lösung wurde mit 4,07 g (50 mmol) Bromwasserstoff in 8,2 g Essigsäure versetzt und bei 10 °C 30 min gerührt. Anschliessend wurden 284,4 g (1,78 mol) Brom innerhalb von 1,5 h zugetropft und das Gemisch noch weitere 1,25 h bei 15 °C gerührt. Das Reaktionsgemisch wurde bei 40 °C/15 mbar auf 300 ml eingeengt und bei 100 °C innerhalb von 1,5 h zu einem Gemisch von 250 ml Schwefelsäure und 18,0 g (1 mol) Wasser getropft, wobei eine lebhafte Gasentwicklung (HBr) stattfand und sich eine Suspension bildete. Diese wurde noch weitere 13 h bei 100 °C gerührt, abgekühlt und filtriert. Der Filterkuchen wurde dreimal mit je 100 ml Aceton gewaschen und anschliessend bei 20 °C/0,1 mbar getrocknet.

Ausbeute: 51,46 g, Gehalt (HPLC) 93,3%, entspr. 84,2% d. Th.

Beispiel 9:

1,3-Cyclobutandion

In 16,4 g eines Ameisensäure-Wasser-Gemischs (95:5) wurden 1,82 g 1,1,3,3-Tetramethoxycyclobutan (Gehalt 96,7%, 10 mmol) gelöst und die Lösung bei 0 °C 2 h gerührt. Anschliessend wurde das Gemisch bei 20 °C/0,1 mbar eingedampft. Der Rückstand (0,66 g) wurde mit 1,5 ml Ethylacetat gewaschen und getrocknet.

Ausbeute: 0,32 g, Gehalt (HPLC) 94,1%, entspr. 35,8% d. Th.
Schmp.: 112 °C (Zers.)

## Patentansprüche

1. 1,3-Cyclobutandion-bisketale der allgemeinen Formel

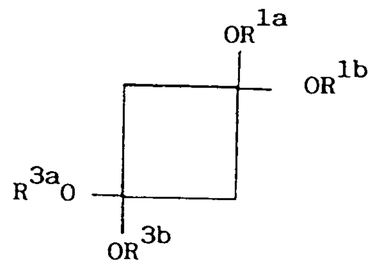

I

worin entweder $R^{1a}$, $R^{1b}$, $R^{3a}$ und $R^{3b}$ gleich sind und jeweils eine lineare oder verzweigte Niederalkylgruppe darstellen,
oder jeweils $R^{1a}$ und $R^{1b}$ sowie $R^{3a}$ und $R^{3b}$ zusammen gleichbedeutend sind und mit den Ketal-Sauerstoffatomen und dem dazwischenliegenden Kohlenstoffatom jeweils einen, gegebenenfalls durch eine oder mehrere Niederalkylgruppen substituierten, fünfgliedrigen oder sechsgliedrigen mit dem Cyclobutanring spiro-verknüpften Ring bilden.

2. Verbindungen nach Patentanspruch 1, dadurch gekennzeichnet, dass $R^{1a}$, $R^{1b}$, $R^{3a}$ und $R^{3b}$ aus der Gruppe Methyl, Ethyl, Propyl und Butyl ausgewählt sind.

3. Verbindungen nach Patentanspruch 1, dadurch gekennzeichnet, dass $R^{1a}$ und $R^{1b}$ sowie $R^{3a}$ und $R^{3b}$ zusammen eine Kohlenstoffkette bilden, die ausgewählt ist aus der Gruppe 1,2-Ethandiyl, 1,2-Propandiyl, 1,3-Propandiyl, 1,2-Butandiyl, 1,3-Butandiyl, 2,3-Butandiyl, 2,2-Dimethyl-1,3-propandiyl und 2,3-Dimethyl-2,3-butandiyl.

4. Verfahren zur Herstellung von 1,3-Cyclobutandion-bisketalen (I) nach Patentanspruch 1, dadurch gekennzeichnet, dass 1,3-Cyclobutandion in Gegenwart eines sauren Katalysators mit einem Orthoameisensäureester der allgemeinen Formel

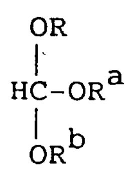

II

worin $R^a$ und $R^b$ die in Formel I für $R^{1a}$ und $R^{1b}$ sowie $R^{3a}$ und $R^{3b}$ genannten Bedeutungen haben und R eine Niederalkylgruppe ist und, sofern $R^a$ und $R^b$ ebenfalls Niederalkylgruppen sind, R, $R^a$ und $R^b$ gleich sind, umgesetzt wird.

5. Verfahren nach Patentanspruch 4, dadurch gekennzeichnet, dass R, $R^a$ und $R^b$ gleich sind und die Umsetzung mit dem Orthoameisensäureester in Gegenwart des entsprechenden Alkohols ROH durchgeführt wird.

6. Verfahren nach Patentanspruch 4 oder 5, dadurch gekennzeichnet, dass als saurer Katalysator ein stark saurer Kationenaustauscher eingesetzt wird.

7. Verfahren zur Herstellung von 1,3-Cyclobutandion-bisketalen (I) nach Patentanspruch 1, dadurch gekennzeichnet, dass 1,3-Cyclobutandion in Gegenwart eines sauren Katalysators in einer ersten Stufe mit einem Orthoameisensäureester der allgemeinen Formel

$HC(OR)_3$ III

worin R eine Niederalkylgruppe ist, zu einem Bisketal der allgemeinen Formel

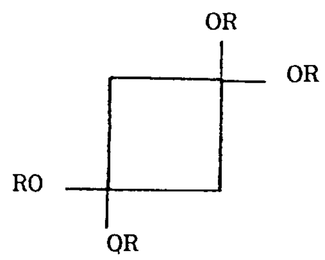

IV

worin R die vorstehend genannte Bedeutung hat, umgesetzt und in einer zweiten Stufe durch Umsetzung mit einem Diol der allgemeinen Formel

HO-Q-OH V

worin Q eine gegebenenfalls durch eine oder mehrere Niederalkylgruppen substituierte Ethandiyl- oder Propandiylgruppe ist, umketalisiert wird.

**8.** Verfahren nach Patentanspruch 7, dadurch gekennzeichnet, dass R Methyl oder Ethyl ist.

**9.** Verfahren nach Patentanspruch 7 oder 8, dadurch gekennzeichnet, dass Q ausgewählt ist aus der Gruppe 1,2-Ethandiyl, 1,2-Propandiyl, 1,3-Propandiyl, 1,2-Butandiyl, 1,3-Butandiyl, 2,3-Butandiyl, 2,2-Dimethyl-1,3-propandiyl- und 2,3-Dimethyl-2,3-butandiyl.

**10.** Verfahren zur Herstellung von 1,3-Cyclobutandion-bisketalen (I) nach Patentanspruch 1, dadurch gekennzeichnet, dass 3-Acetoxy-2cyclobuten-1-on der Formel

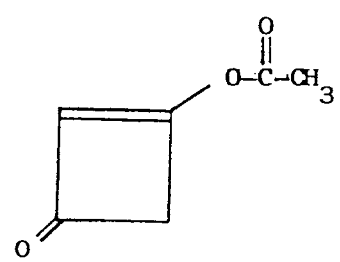

VI

in Gegenwart eines sauren Katalysators mit einem Orthoameisensäureester der allgemeinen Formel

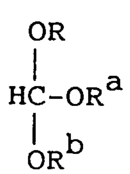

II

worin $R^a$ und $R^b$ die in Formel I für $R^{1a}$ und $R^{1b}$ sowie $R^{3a}$ und $R^{3b}$ genannten Bedeutungen haben und R eine Niederalkylgruppe ist und, sofern $R^a$ und $R^b$ ebenfalls Niederalkylgruppen sind, R, $R^a$ und $R^b$ gleich sind, umgesetzt wird.

**11.** Verfahren nach Patentanspruch 10, dadurch gekennzeichnet, dass das 3-Acetoxy-2-cyclobuten-1-on in Form des bei der Herstellung von Diketen aus Keten anfallenden Destillationsrückstandes eingesetzt wird.

**12.** Verfahren nach Patentanspruch 10 oder 11, dadurch gekennzeichnet, dass R, $R^a$ und $R^b$ gleich sind und die Umsetzung mit dem Orthoameisensäureester in Gegenwart des entsprechenden Alkohols ROH durchgeführt wird.

**13.** Verwendung der 1,3-Cyclobutandion-bisketale gemäss Formel I zur Herstellung von Quadratsäure der Formel

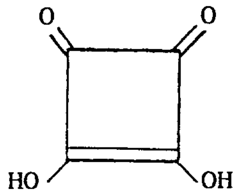

VII

dadurch gekennzeichnet, dass man die 1,3-Cyclobutandion-bisketale zunächst halogeniert und dann zur Quadratsäure hydrolysiert.

**14.** Verwendung nach Patentanspruch 13, dadurch gekennzeichnet, dass man die Halogenierung mit elementarem Chlor oder Brom durchführt.

**15.** Verwendung der 1,3-Cyclobutandion-bisketale gemäss Formel I zur Herstellung und/oder Reinigung von 1,3-Cyclobutandion, dadurch gekennzeichnet, dass man die 1,3-Cyclobutandion-bisketale in Gegenwart einer Säure hydrolysiert.

Europäisches Patentamt

# EUROPÄISCHER RECHERCHENBERICHT

Nummer der Anmeldung

EP 93 10 4703

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X<br>Y | GB-A-1 140 207 (MONTECATINI)<br>* Seite 1, Zeile 41 - Zeile 50 * | 1,3<br>4,6 | C07C43/305<br>C07D493/10<br>C07C49/707<br>C07C49/39<br>C07C41/56<br>C07C45/42<br>C07C45/85<br>//(C07D493/10,317:00,317:00)<br>(C07D493/10,319:00,319:00) |
| Y | 'Methoden der Organischen Chemie (Houben-Weyl), Band E14a/1'<br>1991 , STUTTGART<br>* Seite 280 * | 4,6 | |
| D,A | EP-A-0 442 431 (LONZA)<br>* Ansprüche * | 13-15 | |
| D,A | EP-A-0 444 563 (LONZA)<br>* Ansprüche * | 13-15 | |

RECHERCHIERTE SACHGEBIETE (Int. Cl.5)

C07C
C07D

Der vorliegende Recherchenbericht wurde für alle Patentansprüche erstellt

| Recherchenort | Abschlußdatum der Recherche | Prüfer |
|---|---|---|
| DEN HAAG | 24 JUNI 1993 | WRIGHT M.W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)